# EUROPEAN PATENT SPECIFICATION

(11) **EP 3 178 517 B1**
(45) Date of publication and mention of the grant of the patent: **20.06.2018**
(21) Application number: 17151391.4
(22) Date of filing: 01.02.2016
(51) Int. Cl.: A61M 25/01, A61M 25/10, A61M 25/00, A61B 17/22

(54) **BALLOON CATHETER**
BALLONKATHETER
CATHETER À BALLONNET

(30) Priority: 10.03.2015 JP 2015047146
(43) Date of publication of application: 14.06.2017
(62) Divisional of application: 16153560.4
(73) Proprietor: ASAHI INTECC CO., LTD., Nagoya-shi, Aichi 463-0024 (JP)
(72) Inventor: KATSURADA, Takeharu, Nagoya-shi, Aichi 463-0024 (JP); MOGI, Takeshi, Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 1 787 673
- EP-A1- 2 389 973
- CA-A1- 2 468 108
- JP-A- 2012 020 077

## Description

### TECHNICAL FIELD

The present invention relates to a balloon catheter.

### BACKGROUND ART

Balloon catheters which are used for expanding a stenosis site and the like in a lumen such as a blood vessel are traditionally known.

A balloon catheter mainly comprises a balloon as an expanding object, an outer tube attached to the proximal end portion of the balloon, and an inner tube disposed inside the inner cavity of the outer tube and having a distal end portion attached to the distal end portion of the balloon.

The outer tube is designed for allowing an expansion liquid such as physiological saline to flow through the inner cavity defined between the outer tube and the inner tube in order to expand the balloon. The inner tube is designed for allowing a guide wire to be inserted therethrough in order to guide the balloon catheter to an affected area.

In general, a balloon catheter is used as follows: an operator such as a physician holds the balloon catheter at the proximal portion, and pushes the distal portion thereof into a blood vessel. Therefore, in order to position the balloon in a desired location of an affected area, excellent pushability into the affected area and excellent operativity of the distal portion are required.

As such a balloon catheter, for example, JP 2012-20077 A and EP 1787673 A1 describe a balloon catheter comprising a core wire inserted between a catheter shaft (an outer tube) and an inner shaft (an inner tube) in addition to the configuration described above, wherein the core wire is sandwiched between the outer tube and the inner tube. Another example of a balloon catheter is disclosed in document EP-A-1787673.

According to JP 2012-20077 A, the balloon catheter described in the literature can supposedly improve the pushability of the balloon catheter without the need for a complicated process because the core wire is simply sandwiched between the outer tube and the inner tube.

Further, US 2012/0296367 A1 discloses a balloon catheter comprising a core wire including a proximal section having a substantially uniform outer diameter and a distal tapered section.

US 2012/0253447 A1 discloses a balloon catheter comprising an inner tube, an outer tube and an inflation lumen between the inner tube and the outer tube, wherein the inner tube comprises protruded-depressed portions formed on the outer periphery for preventing the inflation lumen from being blocked or closed-off.

EP 2495006 A1 discloses a balloon catheter comprising an inner tube, an outer tube, an inflation lumen between the inner tube and the outer tube and a core wire having a pushing portion provided at the proximal portion of the core wire.

US 5487757 discloses an electrophysiology catheter comprising a manipulator wire comprising a bulged portion at the distal end.

### SUMMARY OF THE INVENTION

### TECHNICAL PROBLEM

However, the balloon catheter described in JP 2012-20077 A mentioned above has the following problems. Although the pushability of the catheter may be improved, in a case where the proximal end portion of the balloon catheter is pulled, the resulting pull force cannot be transmitted to the balloon catheter through the core wire. Further, in a case where pushing force against the proximal end of the balloon catheter is very strong, the core wire will further move between the outer tube and the inner tube toward the distal end side. Therefore, the core wire will be fixed between the outer tube and the inner tube, interfering with the flexibility of the balloon catheter.

The present invention has been made in order to solve the above problems. An objective of the present invention is to provide a balloon catheter, as described in the claims, in which a function for transmitting force from a core wire to both an outer tube and an inner tube can be maintained even in a case where the force is generated when the proximal end portion of the balloon catheter is pushed and/or pulled, and the core wire is allowed to move appropriately to maintain the flexibility of the balloon catheter even when the balloon catheter bends.

### SOLUTION TO PROBLEM

In order to solve the aforementioned problems, a first aspect of the invention is a balloon catheter comprising a balloon, an outer tube connected to a proximal end of the balloon, an inner tube disposed inside the outer tube and having a distal end connected to the distal end of the balloon, a protruded-depressed portion formed on the outer periphery of the inner tube, and a core wire disposed between the outer tube and the inner tube, wherein the core wire has a bulged portion sandwiched between the outer tube and a depressed portion of the protruded-depressed portion. The protruded-depressed portion may comprise a plurality of protruded portions and a plurality of depressed portions aligned alternatively on the outer periphery of the inner tube in the cross-sectional view parallel to the longitudinal direction of the inner tube. The bulged portion may also be sandwiched between adjacent protruded portions.

Further, the core wire may comprise a distal end portion, and the bulged portion may be connected with the distal end of the distal end portion.

Furthermore, the protruded-depressed portion(s) may be formed by processing the outer periphery of the inner tube. I.e. the protruded-depressed portion(s) may be formed by a part of the inner tube.

Further, a second aspect of the invention is the balloon catheter according to the first aspect of the invention, wherein the protruded-depressed portion is formed by a spirally wound wire of a coil body wound around the outer periphery of the inner tube. In this case, the wire constitutes the protruded portion, and a portion of the outer periphery of the inner tube not covered by the wire constitutes the depressed portion. The wire may have a circular cross-section or a rectangular cross-section.

Further, a third aspect not being part of the invention is the balloon catheter according to the second aspect of the invention, wherein a surface of the coil body is coated with a resin so that the outer periphery of the inner tube forms streamlined protrusions and depressions along the shape of the wire of the coil body.

Further, a fourth aspect not being part of the invention is the balloon catheter according to any one of the first to third aspects of the invention, satisfying Dc > Da > Db wherein Da represents a maximum distance between the inner wall of the outer tube and the depressed portion, Db represents a minimum distance between the inner wall of the outer tube and the protruded portion, and Dc represents the outer diameter of the bulged portion.

### ADVANTAGEOUS EFFECT OF THE INVENTION

The first aspect of the invention is a balloon catheter comprising a balloon, an outer tube connected to a proximal end of the balloon, an inner tube disposed inside the outer tube and having a distal end connected to the distal end of the balloon, a protruded-depressed portion formed on the outer periphery of the inner tube, a core wire disposed between the outer tube and the inner tube, wherein the core wire has a bulged portion sandwiched between the outer tube and a depressed portion of the protruded-depressed portion. Therefore, a force can be transmitted from the core wire to both the outer tube and the inner tube even in a case where the force is generated when the proximal end portion of the balloon catheter is pushed and/or pulled, and the bulged portion of the core wire can be allowed to appropriately move between the protruded-depressed portions to maintain the flexibility of the balloon catheter even when the balloon catheter bends.

Further, the second aspect of the invention is the balloon catheter according to the first aspect of the invention, wherein the protruded-depressed portion is formed by increasing a pitch of a wire of a coil body wound around the outer periphery of the inner tube. Therefore, this simple configuration can allow the force of pushing and/or pulling to be transmitted from the core wire to both the outer tube and the inner tube, and a kink in the inner tube can be prevented in addition to the advantageous effects of the first aspect of the invention.

Further, the third aspect not being part of the invention is the balloon catheter according to the second aspect of the invention, wherein a surface of the coil body is coated with a resin so that the outer periphery of the inner tube forms streamlined protrusions and depressions along the shape of the wire of the coil body. Therefore, the bulged portion can be allowed to move smoothly in addition to the advantageous effects of the second aspect of the invention.

Further, the fourth aspect not being part of the invention is the balloon catheter according to any one of the first to third aspects satisfying Dc > Da > Db wherein Da represents a distance between the inner wall of the outer tube and the depressed portion, Db represents a distance between the inner wall of the outer tube and the protruded portion, and Dc represents the outer diameter of the bulged portion. Therefore, when the proximal end portion of the balloon catheter is pushed and/or pulled, the resulting force can be better transmitted from the core wire to both the outer tube and the inner tube in addition to the advantageous effects of any one of the first to third aspects

### BRIEF DESCRIPTION OF THE DRAWINGS

Fig. 1 shows a schematic overall view of a balloon catheter according to a first embodiment not being part of the present invention.
Fig. 2 shows a partial enlarged view of region A of Fig. 1.
Fig. 3 shows a schematic overall view of a balloon catheter according to a second embodiment.
Fig. 4 shows a partial enlarged view of region B of Fig. 3.
Fig. 5 shows a schematic overall view of a balloon catheter according to a third embodiment.
Fig. 6 shows a partial enlarged view of region C of Fig. 5.
Fig. 7 shows an enlarged view of the main section of a balloon catheter according to a fourth embodiment not being part of the invention.
Fig. 8 shows a diagram illustrating the movement of the bulged portion.

### DESCRIPTION OF EMBODIMENTS

### (First embodiment, which is not part of the present invention)

The configuration of the balloon catheter according to this embodiment will be described below with reference to Figures.

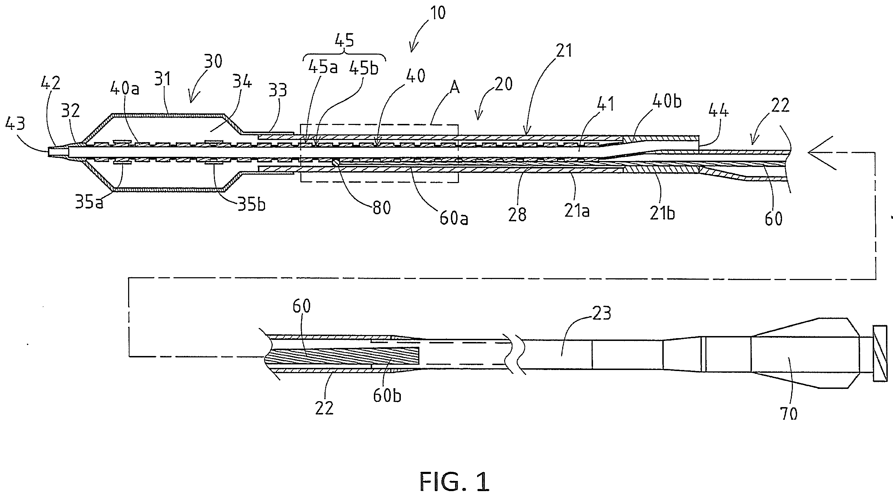

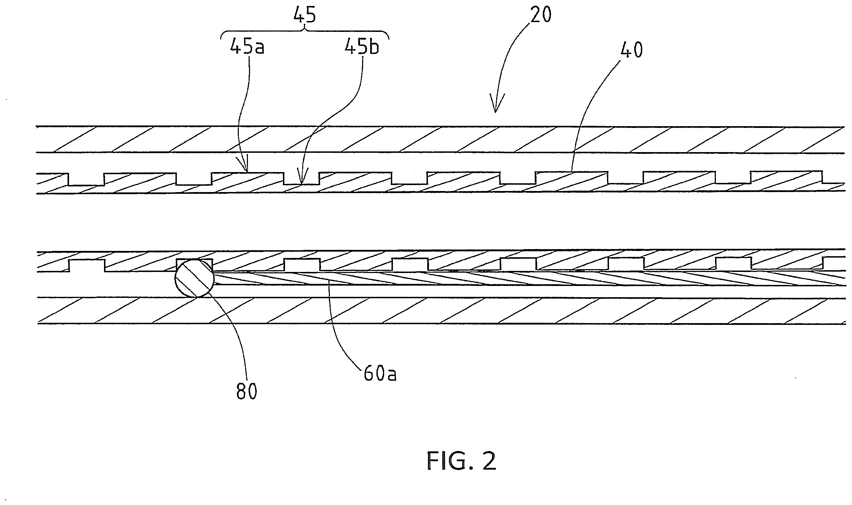

Fig. 1 shows a schematic overall view of a balloon catheter according to a first embodiment of the present invention, and Fig. 2 shows a partial enlarged view of region A of Fig. 1. Note that the left side in Figs. 1 and 2 corresponds to the distal side (the front end side) which is to be inserted into the body, and the right side corresponds to the proximal side (the base end side) which is to be operated by an operator such as a physician.

A balloon catheter 10 shown in Fig. 1 comprises a balloon 30, an outer tube 20 connected to the proximal end portion of the balloon 30, an inner tube 40 inserted inside the outer tube 20 and having a distal end connected with the distal end of the balloon 30, a hypotube 23 connected with the proximal end of the outer tube 20, a core wire 60 inserted between the outer tube 20 and the inner tube 40 and having a proximal end connected with the distal end portion of the hypotube 23 and a connector 70 connected with the proximal end of the hypotube 23.

The balloon 30 comprises an expanding part 31 which expands due to the pressure of a liquid injected inside, a distal attachment part 32 connected with the distal end of a distal end portion 40a of the inner tube 40 and a proximal attachment part 33 connected with the outer periphery of the distal end of the outer tube 20, in which the expanding part 31 can be expanded with the pressure of the liquid injected inside to form an inner cavity 34 and to become a bag-like shape. Further, the inner cavity 34 of the balloon 30 communicates with an inner lumen 28 of the outer tube 20.

The outer tube 20 is a tubular body having a distal end outer tube 21 located on the distal end side, a proximal end portion outer tube 22 located on the proximal end portion side and the inner lumen 28 continuously extending from the distal end outer tube 21 to the proximal end portion outer tube 22. Further, a liquid for expanding the balloon 30 can be allowed to flow through the inner lumen 28 of the outer tube 20.

The distal end outer tube 21 and the proximal end portion outer tube 22 are formed from a resin such as polyamide, polyamide elastomer, polyolefine, polyester and polyester elastomer, for example.

The distal end outer tube 21 comprises a body tube 21a located on the distal end side and a port tube 21b located on the proximal end portion side, in which the proximal attachment part 33 of the balloon 30 is connected with the outer periphery of the distal end of the body tube 21a.

The port tube 21b has an inner cavity in communication with the inner lumen 28 and an opening (a proximal end side opening) 44 in which a proximal end portion 40b of the inner tube 40 described below is opened. Note that the port tube 21b may be formed from a resin harder than that of the body tube 21a.

The inner tube 40 is a tubular body having the distal end portion 40a and the proximal end portion 40b and is inserted coaxially with the outer tube 20 into the inner lumen 28 of the distal end outer tube 21 of the outer tube 20. The inner tube 40 has an inner lumen 41 in which a guide wire not shown in the figure is insertably formed.

Further, a protruded-depressed portion 45 comprising protruded portions 45a and depressed portions 45b which are of spiral or annular shapes in the longitudinal direction is formed on the outer periphery of the inner tube 40 throughout its entire length. In this embodiment, the protruded-depressed portion 45 is formed by processing the outer periphery of the inner tube 40. Note that the inner tube 40 may be formed of the same resin as the distal end outer tube 21 and the proximal end portion outer tube 22.

The inner tube 40 has the distal end portion 40a protruded from the distal end of the distal end outer tube 21, and a tip 42 configured with a soft resin may be attached to the distal end of the distal end portion 40a.

The hypotube 23 is a metal tubular body in which the proximal end portion outer tube 22 is connected to the outer periphery thereof at the distal end portion, and the connector 70 is attached to the proximal end portion. Further, the proximal end portion 60b of the core wire 60 described below is connected with the inside of the distal end of the hypotube 23.

As a material of the hypotube 23, for example, a superelastic alloy such as stainless steel and a Ni-Ti alloy may be used.

The core wire 60 comprises a distal end portion 60a, the proximal end portion 60b and a bulged portion 80 connected with the distal end of the distal end portion 60a and having a larger diameter than the wire diameter of the distal end portion 60a.

The proximal end portion part 60b of the core wire 60 is connected with the inside of the distal end of the hypotube 23 by joining means such as welding, adhesives and the like, and the bulged portion 80 corresponding to the distal end portion of the core wire 60 is sandwiched between the outer tube 20 and the depressed portion 45b of the protruded-depressed portion 45.

The connector 70 is a resin tubular member. When a liquid is supplied from an unshown indeflator attached to the proximal end of the connector 70, the liquid enters the inner cavity 34 of the balloon 30 from the hypotube 23 through the inner lumen 28 to expand the balloon 30.

The tip 42 is a tapered tubular member having an outer diameter gradually decreasing toward the distal end, and has a distal end side opening 43 at the distal end. The tip 42 may be formed of a resin softer than that of the distal end portion 40a of the inner tube 40.

Further, two radiopaque markers 35a and 35b separated by a predetermined distance are attached to the inside of the expanding part 31 of the balloon 30 at the distal end portion 40a of the inner tube 40.

A case where the balloon catheter 10 according to this embodiment is used in a procedure for expanding a stenosis site in the coronary artery of the heart will be described based on the above configuration.

A guide wire is already inserted into the coronary artery of the heart having a stenosis site as a treatment target. First, an operator inserts the proximal end of this guide wire through the distal end side opening 43 of the balloon catheter 10 and allows it to pass through the inner lumen 41 of the inner tube 40, and then retrieves it through the proximal end side opening 44. Subsequently, while holding the guide wire, the proximal end portion of the balloon catheter 10 is pushed toward the distal end side in the axial direction to advance the balloon catheter 10 into the patient's blood vessel.

The pushing force with which an operator pushes is first transmitted to the outer tube 20 and the core wire 60 from the hypotube 23. In this case, the bulged portion 80 disposed at a distal end portion of the core wire 60 is sandwiched between the outer tube 20 and the depressed portion 45b of the protruded-depressed portion 45, and thus the pushing force transmitted to the outer tube 20 and the core wire 60 is transmitted to the inner tube 40 and the outer tube 20.

Further, since the bulged portion 80 is sandwiched, but not fixed, between the outer tube 20 and the depressed portion 45b of the protruded-depressed portion 45, the bulged portion 80 can move to the depressed portion 45b adjacent to the distal end side in a case where the balloon catheter 10 bends toward the side of the core wire 60 due to the serpentine state of a patient's blood vessel. Therefore, the flexibility of the balloon catheter 10 can be maintained. On the other hand, in a case where the balloon catheter 10 bends toward the opposite side of the core wire 60, the bulged portion 80 moves to the depressed portion 45b adjacent to the proximal end portion side. Therefore, the flexibility of the balloon catheter 10 can be maintained.

Supposing that the bulged portion 80 is sandwiched and fixed between the outer tube 20 and the depressed portion 45b of the protruded-depressed portion 45, the balloon catheter 10 is difficult to bend due to the rigidity of the core wire 60 and the like even in a case where the balloon catheter 10 bends toward the side of the core wire 60 or the opposite side of the core wire 60 depending on the serpentine state of a patient's blood vessel. In contrast, the balloon catheter 10 according to this embodiment will bend depending flexibly on the serpentine state of a patient's blood vessel since the rigidity of the core wire 60 will not interfere with the followability of the balloon catheter 10 along the curvature.

Further, the bulged portion 80 moves to the depressed portion 45b adjacent to the distal end side when the pushing force to the core wire 60 exceeds a predetermined value, and thus an excessive force will not be transmitted to the distal end of the balloon catheter 10 such that an operator can safely operate the balloon catheter 10.

Subsequently, an operator positions the balloon 30 at a stenosis site as a target site while observing markers 35a and 35b under radioscopy, and then supplies a liquid from an unshown indeflator connected to the connector 70. At this time, the liquid flows into the inner lumen 28 of the outer tube 20, and flows out from the distal end of the distal end outer tube 21 into the inner cavity 34 of the balloon 30 to expand the balloon 30, which in turn expands the stenosis site at the same time.

After expanding the stenosis site with the balloon 30, the operator discharges the liquid from the balloon 30 with the indeflator. That is, the liquid flows out of the inside of the balloon 30 and is discharged through the inner lumen 28 of the outer tube 20. Then, the balloon catheter 10 is pulled out of the patient's body to complete the procedure.

Pulling force is also first transmitted to the outer tube 20 and the core wire 60 from the hypotube 23 when the operator pulls the balloon catheter 10 out of the patient's body. In this case, the bulged portion 80 disposed at a distal end portion of the core wire 60 is sandwiched between the outer tube 20 and the depressed portion 45b of the protruded-depressed portion 45, and thus the pulling force transmitted to the outer tube 20 and the core wire 60 is transmitted to the inner tube 40 and the outer tube 20, enabling smooth withdrawal of the balloon catheter 10 from the patient's body.

In the case of the balloon catheter 10 according to this embodiment, the following operational effects can be achieved.
(1) The balloon catheter 10 according to this embodiment comprises the balloon 30, the outer tube 20 connected to the proximal end of the balloon 30, the inner tube 40 disposed inside the outer tube 20 and having a distal end connected to the distal end of the balloon 30, the protruded-depressed portion 45 formed on the outer periphery of the inner tube 40, and the core wire 60 disposed between the outer tube 20 and the inner tube 40, wherein the core wire 60 has the bulged portion 80 sandwiched between the outer tube 20 and the depressed portion 45b of the protruded-depressed portion 45. Therefore, a force can be transmitted from the bulged portion 80 of the core wire 60 to both the outer tube 20 and the inner tube 40 even in a case where the force is generated when the proximal end portion of the balloon catheter 10 is pushed and/or pulled.
(2) Even in a case where the balloon catheter 10 bends, the bulged portion 80 of the core wire 60 can be allowed to move appropriately between the depressed portions 45b of the protruded-depressed portion 45, and thus the flexibility of the balloon catheter 10 can be maintained.

### (Second embodiment)

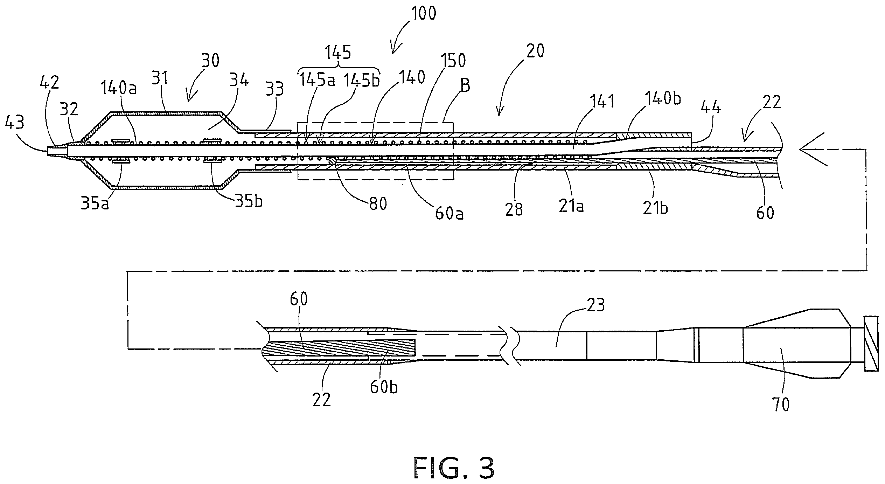

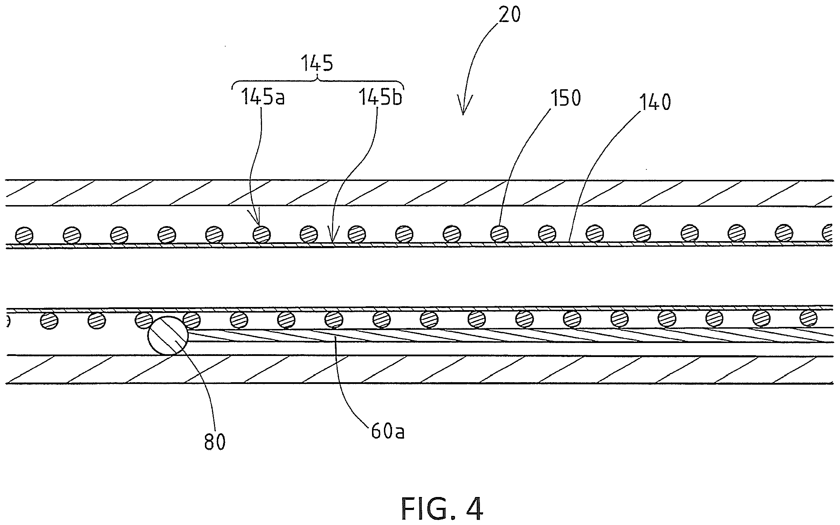

Fig. 3 shows a schematic overall view of a balloon catheter according to a second embodiment. Fig. 4 shows a partial enlarged view of region B of Fig. 3. Note that the left side in Figs. 3 and 4 corresponds to the distal side (the front end side) which is to be inserted into the body, and the right side corresponds to the proximal side (the base end side) which is to be operated by an operator such as a physician.

Further, in Figs. 3 and 4, the same reference numbers are assigned for parts common with the first embodiment.

A balloon catheter 100 shown in Fig. 3 has an inner tube different from that of the balloon catheter 10 according to the first embodiment. That is, an inner tube 140 according to this embodiment has a distal end portion 140a and a proximal end portion 140b, and is a tubular body which is inserted coaxially with the outer tube 20 into the inner lumen 28 of the distal end outer tube 21 of the outer tube 20. The inner tube 140 has an inner lumen 141 in which a guide wire not shown in the figure is insertably formed.

Further, a protruded-depressed portion 145 is formed by spirally winding in the longitudinal direction a metal wire 150 of a coil body with a circular cross section on the outer periphery of the inner tube 140 over its entire length while increasing pitch. The protruded-depressed portion 145 comprises protruded portions 145a and depressed portions 145b on the outer periphery of the inner tube 140 in the cross-sectional view parallel to the longitudinal direction of the inner tube 140. In this embodiment, the wire 150 constitutes the protruded portions 145a and a portion of the outer periphery of the inner tube 140 not covered by the wire 150 constitutes the depressed portions 145b.

Note that in this embodiment, a metal wire with a circular cross section is spirally wound in the longitudinal direction while increasing pitch in order to form the protruded-depressed portion 145. However, a resin wire with a circular cross section may be spirally wound in the longitudinal direction while increasing pitch to form the protruded-depressed portion 145, or a metal wire with a rectangular cross section or a resin wire with a rectangular cross section may be spirally wound in the longitudinal direction while increasing pitch to form the protruded-depressed portion 145.

Further, two or more metal or resin wires with a circular or rectangular cross section may be spirally wound in the longitudinal direction while increasing pitch to form the protruded-depressed portion 145, or one multiple strand wire formed by twisting two or more metal or resin strands with a circular cross section may be spirally wound in the longitudinal direction while increasing pitch to form the protruded-depressed portion 145.

Further, two or more multiple strand wires formed by twisting two or more metal or resin strands with a circular cross section may be spirally wound in the longitudinal direction while increasing pitch to form the protruded-depressed portion 145.

The bulged portion 80 disposed at a distal end portion of the core wire 60 is sandwiched between the outer tube 20 and the depressed portion 145b of the protruded-depressed portion 145.

In a case where the balloon catheter 100 according to this embodiment having a configuration as described above is used in a procedure for expanding a stenosis site in the coronary artery of the heart, a pushing and/or pulling force can be transmitted from the bulged portion 80 of the core wire 60 to both the outer tube 20 and the inner tube 140, and a kink in the inner tube 140 can be prevented by utilizing this simple configuration of the protruded-depressed portion 145 formed by increasing a pitch of the wire 150 of the coil body wound around the outer periphery of the inner tube 140.

### (Third embodiment)

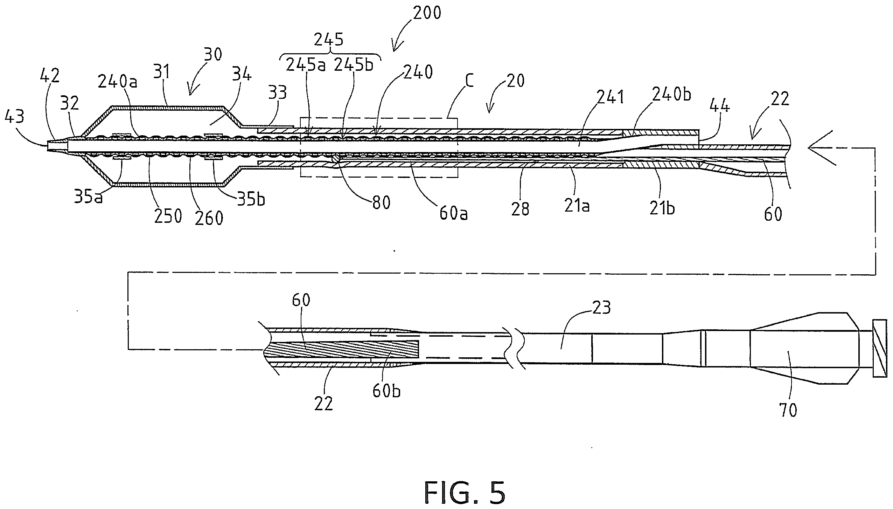

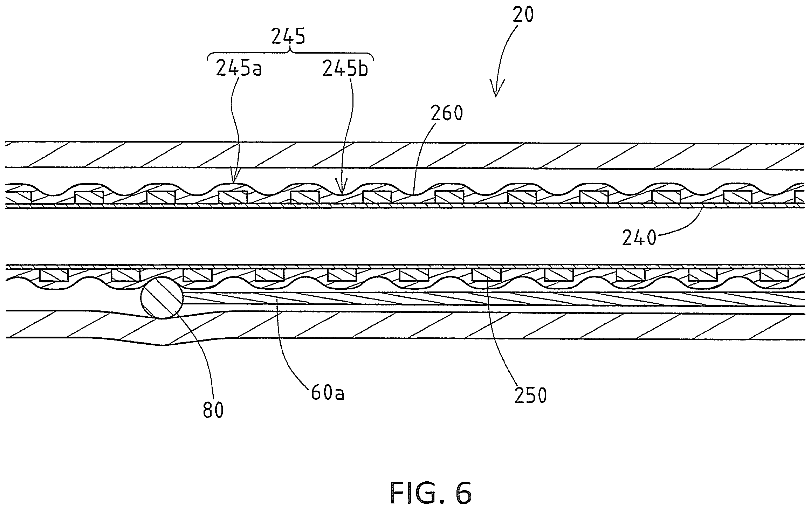

Fig. 5 shows a schematic overall view of a balloon catheter according to a third embodiment. Fig. 6 shows a partial enlarged view of region C of Fig. 5. Note that the left side in Figs. 5 and 6 corresponds to the distal side (the front end side) which is to be inserted into the body, and the right side corresponds to the proximal side (the proximal end side) which is to be operated by an operator such as a physician.

Further, in Figs. 5 and 6, the same reference numbers are assigned for parts common with the first embodiment.

A balloon catheter 200 shown in Fig. 5 has an inner tube different from that of the balloon catheter 10 according to the first embodiment. That is, a metal wire 250 with a rectangular cross section is spirally wound in the longitudinal direction on the outer periphery of an inner tube 240 according to this embodiment over the entire length of the inner tube 240 while increasing pitch, and a surface of the metal wire 250, which constituents of a coil body, is coated with a resin 260 so that an outer periphery of the inner tube 240 forms streamlined protrusions and depressions along the shape of the metal wire with a rectangular cross section to form a protruded-depressed portion 245 comprising protruded portions 245a and depressed portions 245b.

As a resin material for coating so that the outer periphery of the inner tube 240 forms streamlined protrusions and depressions along the shape of the metal wire with a rectangular cross section, a resin such as polyamide, polyamide elastomer, polyolefine, polyester and polyester elastomer, for example, may be used.

Note that in this embodiment, the metal wire 250 with a rectangular cross section is spirally wound in the longitudinal direction while increasing pitch in order to form the protruded-depressed portion 245. However, a resin wire with a rectangular cross section may be spirally wound in the longitudinal direction while increasing pitch to form the protruded-depressed portion 245, or a metal wire with a circular cross section or a resin wire with a circular cross section may be spirally wound in the longitudinal direction while increasing pitch to form the protruded-depressed portion 245.

Further, two or more metal or resin wires with a circular or rectangular cross section may be spirally wound in the longitudinal direction while increasing pitch to form the protruded-depressed portion 245, or one multiple strand wire formed by twisting two or more metal or resin strands with a circular cross section may be spirally wound in the longitudinal direction while increasing pitch to form the protruded-depressed portion 245.

Further, two or more multiple strand wires formed by twisting two or more metal or resin strands with a circular cross section may be spirally wound in the longitudinal direction while increasing pitch to form the protruded-depressed portion 245.

The bulged portion 80 disposed at a distal end portion of the core wire 60 is sandwiched between the outer tube 20 and the depressed portion 245b of the protruded-depressed portion 245.

In a case where the balloon catheter 200 according to this embodiment having a configuration as described above is used in a procedure for expanding a stenosis site in the coronary artery of the heart, the bulged portion 80 can move smoothly, in addition to the advantageous effects of the second aspect of the invention, because the surface of the metal wire 250 of the coil body is coated with the resin 260 so that the outer periphery of the inner tube 240 forms streamlined protrusions and depressions along the shape of the metal wire with a rectangular cross section to form the protruded-depressed portion 245 comprising protruded portions 245a and depressed portions 245b.

### (Fourth embodiment, which is not part of the present invention)

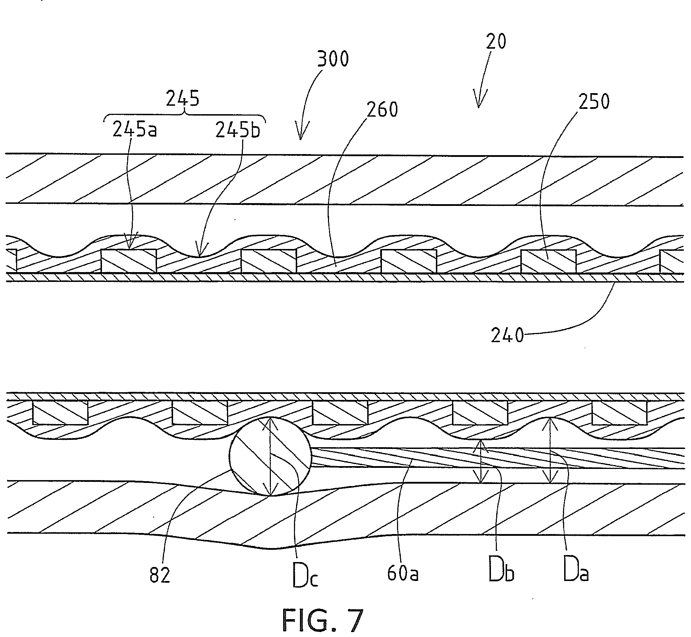

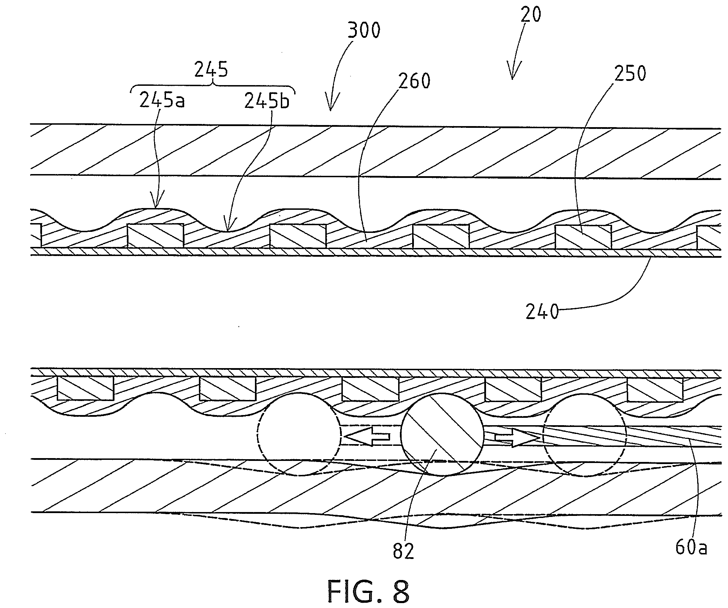

Fig. 7 shows an enlarged view of the main section of a balloon catheter 300 according to a fourth embodiment. Fig. 8 shows a diagram illustrating the movement of a bulged portion 82. This embodiment satisfies Dc > Da > Db wherein Da represents a distance between the inner wall of the outer tube 20 and the depressed portion 245b, Db represents a distance between the inner wall of the outer tube 20 and the protruded portion 245a, and Dc represents the outer diameter of the bulged portion 82.

The balloon catheter 300 according to this embodiment satisfies Dc > Da > Db wherein Da represents a maximum distance between the inner wall of the outer tube 20 and the depressed portion 245b, Db represents a minimum distance between the inner wall of the outer tube 20 and the protruded portion 245a, and Dc represents the outer diameter of the bulged portion 82. This is, Da represents the distance between the inner wall of the outer tube 20 and the bottom of the depressed portion 245b, and Db represents the distance between the inner wall of the outer tube 20 and the top of the protruded portion 245a. Therefore, the pushability from the core wire 60 to both the inner tube 240 and the outer tube 20 can further be improved.

Further, as shown in Fig. 8, when the balloon catheter 300 bends toward the side of the core wire 60a depending on the serpentine state of a patient's blood vessel, the bulged portion 82 can move to the depressed portion 245b adjacent to the distal end side (the left side in the figure) to maintain the flexibility of the balloon catheter 300. On the other hand, when the balloon catheter 300 bends toward the opposite side of the core wire 60a, the bulged portion 82 can move to the depressed portion 245b adjacent to the proximal end side (the right side in the figure) to maintain the flexibility of the balloon catheter 300.

Note that although Fig. 8 describes the movement of the bulged portion 82 to the depressed portion 245b adjacent to the distal end side (the left side in the figure) and the proximal end side (the right side in the figure), it is apparent that the bulged portion 80 in the first to third embodiments can also move in a similar fashion.

Note that although the balloon catheters 10, 100, 200, 300 according to the first to fourth embodiments have been described in the context of use for the treatment of a blood vessel of the heart, they may be used in various procedures such as those for expanding a blood vessel of a lower limb and for expanding a dialysis shunt. Further, the balloon catheters 10, 100, 200, 300 according to the first to fourth embodiments has the bulged portion 80, 82 provided at a distal end of the core wire 60. This present is not limited to the structure, the bulged portion 80, 82 may be provided at the middle of the core wire 60.

### Description of Symbols

10, 100, 200, 300 balloon catheter
20 outer tube
28 inner cavity of outer tube
30 balloon
34 inner cavity of balloon
40, 140, 240 inner tube
45, 145, 245 protruded-depressed portion
45a, 145a, 245a protruded portion
45b, 145b, 245b depressed portion
60 core wire
60a distal end portion of core wire
60b proximal end portion of core wire
150, 250 metal wire

## Claims

1. A balloon catheter (100, 200, 300) comprising:
a balloon (30);
an outer tube (20) connected to a base end of the balloon (30);
an inner tube (140, 240) disposed inside the outer tube (20) and having a front end connected to a front end of the balloon (30);
a core wire (60) disposed between the outer tube (20) and the inner tube (140, 240); and
a protruded-depressed portion (145, 245) formed on an outer periphery of the inner tube (140, 240),
**characterized in that**
the protruded-depressed portion (145, 245) comprises protruded portions (145a, 245a) formed by a spirally wound wire (150, 250) of a coil body, and depressed portions (145b, 245b) constituted of a portion of the outer periphery of the inner tube (140, 240) not covered by the spirally wound wire (150, 250), and
the core wire (60) has a bulged portion (80, 82) sandwiched between the outer tube (20) and a depressed portion (145b, 245b) of the protruded-depressed portion (145, 245).

2. The balloon catheter (100, 200, 300) according to claim 1, wherein the bulged portion (80, 82) is disposed at a distal end of the core wire (60).

3. The balloon catheter (100, 200, 300) according to claim 1 or 2, wherein the wire (150, 250) has a circular cross section or a rectangular cross section.

## Patentansprüche

1. Ballonkatheter (100, 200, 300) mit:
einem Ballon (30);
einer mit einem Basisende des Ballons (30) verbundenen Außenröhre (20);
einem in der Außenröhre (20) angeordneten Innenrohr (140, 240), deren vorderes Ende mit einem vorderen Ende des Ballons (30) verbunden ist;
einem zwischen der Außenröhre (20) und der Innenröhre (140, 240) angeordneten Kerndraht (60); und
einem am Außenumfang der Innenröhre (140, 240) geformten Abschnitt (145, 245) mit Vorsprüngen und Vertiefungen, **dadurch gekennzeichnet, dass**
der Abschnitt (145, 245) mit Vorsprüngen und Vertiefungen Vorsprünge (145a, 245a), die aus einem spiralgewickelten Draht (150, 250) eines Wicklungskörpers geformt sind, und Vertiefungen (145b, 245b), die aus einem von dem spiralgewickelten Draht (150, 250) nicht verdeckten Abschnitt des Außenumfangs der Innenröhre (140, 240) gebildet sind, aufweist, und
der Kerndraht (60) einen zwischen der Außenröhre (20) und einer Vertiefung (145b, 245b) des Abschnitts (145, 245) mit Vorsprüngen und Vertiefungen eingezwängten Wulst (80, 82) hat.

2. Ballonkatheter (100, 200, 300) nach Anspruch 1, wobei der Wulst (80, 82) an einem distalen Ende des Kerndrahts (60) angeordnet ist.

3. Ballonkatheter (100, 200, 300) nach Anspruch 1 oder 2, wobei der Draht (150, 250) einen kreisförmigen oder rechteckigen Querschnitt hat.

## Revendications

1. Cathéter à ballonnet (100, 200, 300) comprenant :
un ballonnet (30) ;
un tube extérieur (20) raccordé à une extrémité de base du ballonnet (30) ;
un tube intérieur (140, 240) disposé à l'intérieur du tube extérieur (20) et ayant une extrémité avant raccordée à une extrémité avant du ballonnet (30) ;
un fil central (60) disposé entre le tube extérieur (20) et le tube intérieur (140, 240) ; et
une partie en saillie renfoncée (145, 245) formée sur une périphérie extérieure du tube intérieur (140, 240),
**caractérisé en ce que**
la partie en saille renfoncée (145, 245) comprend des parties en saillie (145a, 245a) formées par un fil enroulé en spirale (150, 250) d'un corps de bobine, et des parties renfoncées (145b, 245b) constituées d'une partie de la périphérie extérieure du tube intérieur (140, 240) non couverte par le fil enroulé en spirale (150, 250), et
le fil central (60) a une partie renflée (80, 82) prise en sandwich entre le tube extérieur (20) et une partie renfoncée (145b, 245b) de la partie en saillie renfoncée (145, 245).

2. Cathéter à ballonnet (100, 200, 300) selon la revendication 1, dans lequel la partie renflée (80, 82) est disposée au niveau d'une extrémité distale du fil central (60).

3. Cathéter à ballonnet (100, 200, 300) selon la revendication 1 ou 2, dans lequel le fil (150, 250) a une section transversale circulaire ou une section transversale rectangulaire.
